Europäisches Patentamt

(19) European Patent Office    (11) Publication number: **0 056 234**

Office européen des brevets    **B1**

(12) **EUROPEAN PATENT SPECIFICATION**

(45) Date of publication of patent specification: **02.01.85**    (51) Int. Cl.⁴: **A 61 B 1/12,** A 61 M 1/00,
F 16 K 11/02

(21) Application number: **82100021.3**

(22) Date of filing: **05.01.82**

(54) **Endoscope.**

(30) Priority: **14.01.81 JP 4292/81**

(43) Date of publication of application:
**21.07.82 Bulletin 82/29**

(45) Publication of the grant of the patent:
**02.01.85 Bulletin 85/01**

(84) Designated Contracting States:
**AT BE CH DE FR GB IT LI NL SE**

(56) References cited:
**GB-A-1 088 565**
**US-A-3 469 582**
**US-A-3 903 877**
**US-A-4 062 360**
**US-A-4 240 411**

(73) Proprietor: **OLYMPUS OPTICAL CO., LTD.**
**43-2, 2-chome, Hatagaya Shibuya-ku**
**Tokyo 151 (JP)**

(72) Inventor: **Kubokawa, Hiroaki**
**4-22-13, Owada-machi**
**Hachioji-shi Tokyo (JP)**

(74) Representative: **Lins, Edgar, Dipl.-Phys. et al**
**Patentanwälte Gramm + Lins Theodor-Heuss-**
**Strasse 2**
**D-3300 Braunschweig (DE)**

Courier Press, Leamington Spa, England.

## Description

The present invention relates to an endoscope provided with a suction changeover means.

Generally, the endoscope has a suction path through which dirt and mucus in the body cavity are sucked, and a suction changeover means arranged at the endoscope operating section to selectively communicate and shut the suction path. One of conventional suction changeover means has a cylinder arranged on the way of suction path and opened to the outside atmosphere at one end thereof. In the case of endoscope provided with such suction changeover means, negative pressure acts on the suction path so as to allow suction in the body cavity to be achieved only when the opening of cylinder is closed by a finger. However, the suction path is usually communicated with atmosphere in this case and gas is therefore allowed to escape outside the body through the suction path when it is necessary that gas is fed into the body cavity to expand the body cavity ready for observation and treatment. Another measure is to arrange a piston in this cylinder and only when this piston is pushed by a finger, the suction path is communicated with a suction source through a communicating path provided in the piston. However, this makes the endoscope complicate in construction and difficult to manufacture with the problem of increasing the number of parts needed.

A similar arrangement is known from US—A—3,903,877. A cylinder having a plurality of communication chambers is used for switching over a feeding path for a nozzle of an endoscope being disposed near the view window at the distal end of said endoscope. Corresponding to the position of the cylinder water or air is pumped to the nozzle which serves for cleaning and drying the view window.

The present invention is intended to eliminate the mentioned drawbacks of the known suction change-over means and the object of the present invention is, therefore, to provide an endoscope simple in construction and capable of allowing gas in the body cavity to be discharged only when pressure in the body cavity becomes higher than a predetermined value and being easily cleaned and cleanly used.

This invention can be more fully understood from the following detailed description when taken in conjunction with the accompanying drawings, in which:

Fig. 1 shows one embodiment of an endoscope according to the present invention in which a suction changeover means is under an operation;

Fig. 2 is a sectional view showing the endoscope shown in Fig. 1 in which the suction changeover means is under another operation;

Fig. 3 schematically shows another embodiment of an endoscope according to the present invention; and

Figs. 4 and 5 are sectional views showing variations of suction changeover means.

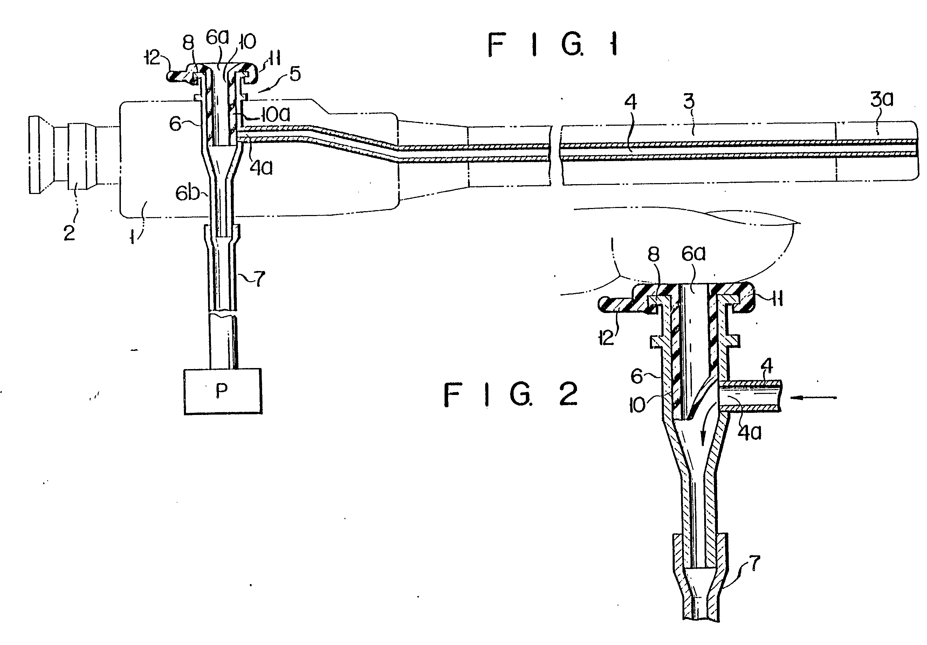

A first embodiment of the present invention will be now described with reference to Figs. 1 and 2. Reference numeral 1 represents an operating section of an endoscope where are arranged an ocular optical system 2, an angle operating knob (not shown) and the like. An elongated insertion section 3 inserted into the body cavity is attached at the base end thereof to the operating section 1. An image guide, a light guide, an air supply pipe (not shown) and a suction path 4 are inserted through the insertion section 3. One end of suction path 4 is opened at the side wall of a cylinder which will be described later while the other end thereof at the end face of foremost end 3a of insertion section 3.

A suction changeover means or mechanism 5 is arranged at the operating section 1 as will be now described. A cylinder 6 is fixed at the operating section 1. One end of cylinder 6 is opened outside as a pressure discharging opening 6a and the other end thereof serves as a connecting opening 6b to which a suction pipe 7 is connected. To the suction pipe 7 is freely detachably connected a suction source or vacuum pump P, for example. Around the circumference of pressure discharging opening 6a is formed a collar 8 for stopping a cylindrical valve 10. One end of suction path 4 is opened as an opening 4a at the side wall of cylinder 6.

The cylindrical valve 10 is fitted into the cylinder 6. The cylindrical valve 10 has an outer diameter substantially same as the inner diameter of cylinder 6, and a body 10a whose both ends are opened and which is made of resilient material such as rubber and synthetic resin. The outer surface of the body 10a closely contacts with the inner surface of cylinder 6 and resiliently closes the opening 4a of suction path 4 by one end outer surface thereof to shut the communication between the suction path 4 and the suction pipe 7. Around the circumference of opened upper end of body 10a is formed a recess opened underside and having an L-shaped cross-section. A stopper collar portion 11 is formed integral to the recess and engaged with the collar fitting portion 8 when the collar fitting portion 8 is fitted in the recess. A finger portion 12 is projected horizontally from a part of stopper collar portion 11. When the finger portion 12 is lifted holding it by fingers to take off the stopper collar portion 11 from the collar fitting portion 8, therefore, the cylindrical valve 10 can be easily detached from the cylinder 6.

The suction changeover means thus arranged leaves the suction pipe 7 communicating with outside atmosphere through the pressure discharging opening 6a when the pressure discharging opening 6a is not closed by a finger. Therefore, no negative pressure caused by the suction source P acts on the suction path 4, thus preventing suction in the body cavity from being carried out. When the pressure discharging opening 6a is closed by finger as shown in Fig. 2, that part of cylindrical valve 10 which faces the opening 4a is resiliently pushed by the pressure difference caused at the opening 4a and the connecting opening 6b thanks to negative pressure in the suction pipe 7, so that the suction path 4 is communicated with the suction pipe 7. Dirt, mucus, cleaning water and the like in the body cavity can be therefore sucked through the suction path 4 and collected into a collecting bottle or the like through the suction pipe 7.

In the case where gas such as air is fed to expand the body cavity ready for observation and treatment, a gas supply means (not shown) belonging to the endoscope is rendered operative to feed gas under pressure into the body cavity through a gas supply pipe. Since the opening 4a of suction path 4 is resiliently closed by the cylindrical valve 10, gas in the body cavity is prevented from leaking out. However, when pressure in the body cavity abnormally becomes high, the cylindrical valve 10 is automatically pushed, thus allowing gas in the body cavity to be discharged. Abnormal expansion of body cavity can be therefore prevented keeping the patient safe and free from pain.

According to the embodiment of the present invention there is provided the suction changeover means as described above and comprising the combination of simple-shaped cylinder 6 having the pressure discharging opening 6a, and cylindrical valve 10 made of resilient material. This suction changeover means is simple in construction and easy to manufacture and can prevent gas in the body cavity from leaking out. In addition, the cylindrical valve 10 can be easily detached from the cylinder 6 when the stopper collar portion 11 is taken off from the collar fitting portion 8. These simple cylinder-shaped valve 10 and cylinder 6 allow therefore their cleaning to be easily carried out ready for a subsequent clean use.

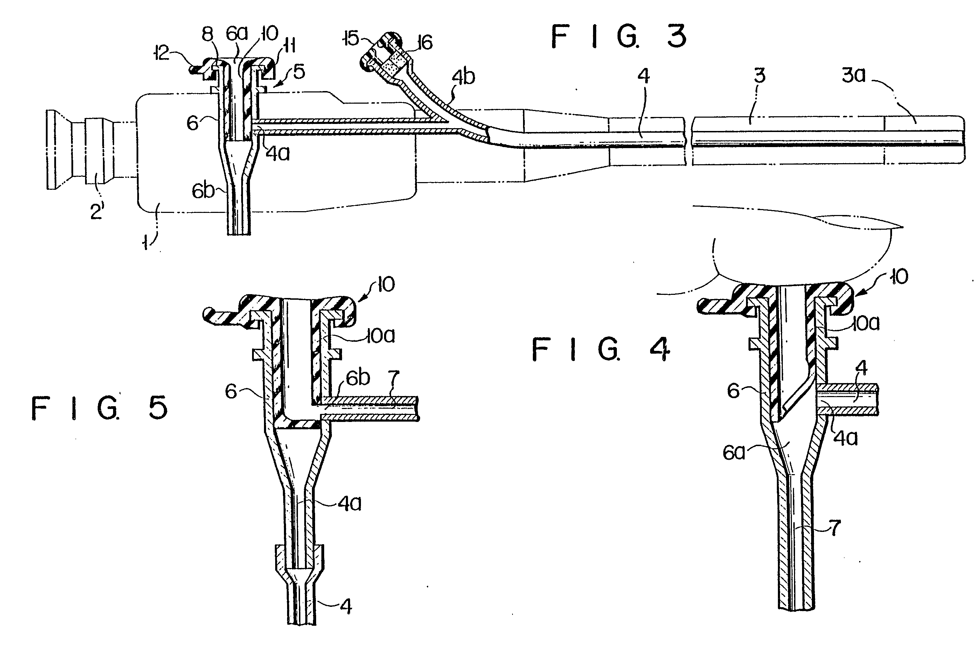

Fig. 3 shows a second embodiment of the present invention in which the suction path 4 is divided on the way thereof to form a branched path 4b having an opening 15 at the foremost end thereof. This second embodiment allows treatment tools such as biopsy forceps to be inserted into the body cavity through the opening 15, brached path 4b and suction path 4. An airtight packing 16 is arranged in the branched path 4b adjacent to the opening 15 thereof. As shown in Fig. 3, the packing 16 fluidly separates the opening 15 from the suction path 4 but allows treatment tools to be inserted into the branched path. Other arrangement of this second embodiment is same as that of first embodiment and same parts as those in the first embodiment are therefore represented by same reference numerals and description on these parts will be omitted.

That portion of cylindrical valve which serves to open and close the opening 4a of suction path may be formed thinner to be easily resiliently deformed.

The body 10a of cylindrical valve 10 is formed to have a uniform thickness as a whole, but that portion thereof which faces the opening 4a may be made thinner as shown in Fig. 4 to achieve easy resilient deformation. That portion of body 10a which serves to perform valve function may be made as a member different from the other portion.

A variation of suction changeover means will be now described referring to Fig. 5. Those parts which achieve same functions as those in above-mentioned embodiments will be represented by same reference numerals and description on those parts will be omitted.

The cylinder 6 has at the lower end thereof an opening portion 4a to which a suction path 4 is connected, and at the circumferential wall a connecting opening 6b to which a suction pipe 7 is connected. The cylindrical body 10a of valve 10 includes a bottom wall and a circumferential wall, which are formed integral to each other and made of resilient material such as synthetic resin. The bottom wall of cylindrical body 10a is formed so as to close the opening portion 4a and at the circumferential wall thereof is formed a hole for communicating the through-hole of cylindrical body 10a with the connecting opening 6b.

When the suction changeover means thus arranged is under the condition shown in Fig. 5, the suction pipe 7 is communicated with atmosphere through the connecting opening 6b, through-hole of cylindrical body 10a and pressure discharging opening 6a, so that no suction function is achieved. When the pressure discharging opening 6a is closed by finger to shut the through-hole of cylindrical body 10a from atmosphere, that portion of bottom wall of cylindrical body 10a which is adjacent to the connecting opening is drawn and deformed toward the connecting opening thanks to negative pressure caused at the connecting opening, so that the interception between the connecting opening 7 and the opening portion 4a is released. This enables the same suction function as in above-mentioned ambodiments to be achieved through the suction path.

As described above, the endoscope of the present invention includes the valve and cylinder arranged in such a way that the valve is fitted into the cylinder and the opening to the suction path is closed from inside using the resilient force of cylindrical valve. The endoscope is therefore simple in construction, small in number of parts needed and easy to manufacture. In addition, when pressure in the body cavity increases abnormally, the suction changeover means also functions as a safe valve to discharge said abnormally-increased pressure and gas supply to the body cavity can be therefore achieved safely. Further, since the cylindrical valve can be detached from the cylinder, these members can be easily cleaned and used cleanly, thus achieving high practical effect.

## Claims

1. An endoscope connected to a suction source comprising:

an operating section (1);

an insertion section (3) having at one end thereof a foremost end portion inserted into a body cavity and being connected at the other end thereof to the operating section, said insertion section housing a suction path (4) whose one end is opened at the foremost end thereof and whose other end is projected into the operating section; and

a suction changeover means (5) arranged at the operating section, said suction changeover means (5) including a cylinder (6) having a connecting opening (6b) connected to the suction source (P), a pressure discharging opening (6a) opened to the outside atmosphere and an opening (4a) connected to the other end of said suction path, and a valve (10) having a body (10a) fitted into said cylinder and a through-hole formed in the body (10a) to usually communicate the pressure discharging opening (6a) with the connecting opening (6b), characterized in that said body (10a) is fixed to said cylinder (6), said body (10a) resiliently shutting the communication between the opening (4a) and the discharging opening (6a) and between the opening (4a) and the connecting opening (6b) when the pressure discharging opening (6a) is communicated with atmosphere, but said body (10a) being resiliently deformed due to negative pressure caused by said suction source in the cylinder (6) to communicate the opening (4a) with the suction path when the pressure discharging opening (6a) is shut off from atmosphere.

2. An endoscope according to claim 1 wherein the body (10a) of said valve (10) has the through-hole and a circumferential wall defining the through-hole and is of cylindrical shape concentrically and detachably fitted into the cylinder (6).

3. An endoscope according to claim 2, wherein the cylindrical body (10a) of said valve (10) has an outer diameter substantially same as the inner diameter of said cylinder (6) and is closely contacted at the outer surface thereof with the inner surface of said cylinder.

4. An endoscope according to claim 3 wherein the cylinder (6) has the pressure discharging opening (6a) formed at one end thereof, the connecting opening (6b) formed at the other end thereof, and a circumferential wall by which the opening is formed, and the cylindrical body of said valve is fitted into the cylinder (6) in such a way that a part of circumferential wall of said cylindrical body closes the opening (4a) of said cylinder.

5. An endoscope according to claim 4 wherein that at least part of said cylindrical body which closes the opening (4a) of said cylinder (6) is made of resilient material.

6. An endoscope according to claim 5 wherein said valve (10) has a means (11) for engaging said valve with the cylinder (6) to fix the former (10) to the latter (6).

7. An endoscope according to claim 6 wherein said valve (10) is made of resilient material as a single unit.

8. An endoscope according to claim 7 wherein the circumferential wall of said cylindrical body has a uniform thickness.

9. An endoscope according to claim 7 wherein that part of said cylindrical body which closes the opening (4a) of said cylinder is made thinner.

10. An endoscope according to claim 3 wherein said cylinder (6) has the pressure discharging opening (6a) formed at one end thereof,*) and the circumferential wall by which the connecting opening is formed, and said cylindrical body (10a) has a bottom wall for closing the opening (4a) of said cylinder and a hole formed in the circumferential wall thereof to communicate the connecting opening (6b) with the through-hole.

11. An endoscope according to claim 10 wherein the bottom and circumferential walls of said cylindrical body (10a) are formed integral to each other and made of resilient material.

12. An endoscope according to any one of the preceding claims wherein said suction path (4) has a branched path (4b) and said branched path (4b) has an opening (15) through which treatment tools are inserted.

13. An endoscope according to claim 12 wherein a packing (16) is arranged in the branched path (4b) to shut the fluid communication of the suction path from the treatment tool inserting opening.

## Revendications

1. Endoscope raccordé à une source d'aspiration comprenant:

une section de manoeuvre (1),

une section d'introduction (3) comportant, à une extrémité, une partie d'extrémité antérieure introduite dans une cavité du corps et raccordée à son autre extrémité à la section de manoeuvre, la section d'introduction contenant un trajet d'aspiration (4) dont une extrémité s'ouvre à son extrémité antérieure et dont l'autre extrémité s'étend dans la section de manoeuvre, et

un dispositif de commutation d'aspiration (5) disposé au niveau de la section de manoeuvre, ce dispositif de commutation d'aspiration (5) comprenant un cylindre (6) comportant un orifice de raccordement (6b) raccordé à la source d'aspiration (P), un orifice d'évacuation de la pression (6a) qui s'ouvre dans l'atmo-

* the opening (4a) formed at the other end thereof.

sphère extérieure et un orifice (4a) raccordé à l'autre extrémité du trajet d'aspiration, ainsi qu'une valve (10) comportant un corps (10a) qui s'ajuste dans le cylindre et un passage de traversée ménagé dans le corps (10a) pour habituellement faire communiquer l'orifice d'evacuation de la pression (6a) avec l'orifice de raccordement (6b), caractérisé en ce que le corps (10a) est fixé au cylindre (6), ce corps (10a) obturant élastiquement la communication entre l'orifice (4a) et l'orifice d'évacuation (6a) et entre l'orifice (4a) et l'orifice de raccordement (6b) lorsque l'orifice d'évacuation de la pression (6a) est mis en communication avec l'atmosphère, mais étant déformé élastiquement par suite de la dépression causée par la source d'aspiration dans le cylindre (6) afin de faire communiquer l'orifice (4a) avec le trajet d'aspiration lorsque l'orifice d'évacuation de la pression (6a) est isolé de l'atmosphère.

2. Endoscope suivant la revendication 1, dans lequel le corps (10a) de la valve (10) comprend le passage de traversée et une paroi circonférentielle délimitant ce passage de traversée, est de forme cylindrique et est engagé concentriquement et de manière amovible dans le cylindre (6).

3. Endoscope suivant la revendication 2, dans lequel le corps cylindrique (10a) de la valve 10 a un diamètre extérieur en substance égal au diamètre intérieur du cylindre (6) et est en contact étroit, au niveau de sa surface externe, avec la surface interne du cylindre.

4. Endoscope suivant la revendication 3, dans lequel le cylindre (6) comprend l'orifice d'évacuation de la pression (6a) ménagé dans une de ses extrémités, l'orifice de raccordement (6b) ménagé dans l'autre de ses extrémités et une paroi circonférentielle par laquelle l'orifice est formé, et le corps cylindrique de la valve est engagé dans le cylindre (6) d'une manière telle qu'une partie de la paroi circonférentielle du corps cylindrique obture l'orifice (4a) du cylindre.

5. Endoscope suivant la revendication 4, caractérisé en ce qu'au moins une partie du corps cylindrique qui ferme l'orifice (4a) du cylindre (6) est faite d'une matière élastique.

6. Endoscope suivant la revendication 5, dans lequel la valve (10) comporte un moyen (11) destiné à engager la valve avec le cylindre (6) pour fixer cette valve (10) à ce cylindre (6).

7. Endoscope suivant la revendication 6, dans lequel la valve (10) est faite d'une matière élastique et est d'une seule pièce.

8. Endoscope suivant la revendication 7, dans lequel la paroi circonférentielle du corps cylindrique a une épaisseur uniforme.

9. Endoscope suivant la revendication 7, dans lequel la partie du corps cylindrique qui ferme l'orifice (4a) du cylindre est amincie.

10. Endoscope suivant la revendication 3, dans lequel le cylindre (6) présente l'orifice d'évacuation de la pression (6a) ménagé dans une de ses extrémités, l'orifice (4a) ménagé dans l'autre de ses extrémités, et la paroi circonférentielle par laquelle l'orifice de raccordement est formé, et le corps cylindrique (10a) comporte une paroi inférieure destinée à obturer l'orifice (4a) du cylindre et une lumière ménagée dans la paroi circonférentielle pour faire communiquer l'orifice de raccordement (6b) avec le passage de traversée.

11. Endoscope suivant la revendication 10, dans lequel la paroi inférieure et la paroi circonférentielle du corps cylindrique (10a) sont d'une pièce l'une avec l'autre et sont faites d'une matière élastique.

12. Endoscope suivant l'une quelconque des revendications précédentes, dans lequel le trajet d'aspiration (4) comporte un trajet de branchement (4b) qui présente un orifice (15) par lequel des instruments sont introduits.

13. Endoscope suivant la revendication 12, dans lequel un bourrage (16) est disposé dans le trajet de branchement (4b) pour couper la communication de fluide du trajet d'aspiration à partir de l'orifice d'introduction des instruments.

**Patentansprüche**

1. Mit einer Absaugquelle verbundenes Endoskop mit:

einem Bedienteil (1),

einem Einführungsteil (3), das an einem Ende einen vorderen, in eine Körperhöhle eingeführten Endabschnitt aufweist und an seinem anderen Ende mit dem Bedienteil verbunden ist und das einen Absaugkanal (4) umschließt, dessen eines Ende am vorderen Ende offen ist und dessen anderes Ende in das Bedienteil hineinragt, und

einer am Bedienteil angebrachten Absaug-Umschalteinrichtung (5) mit einem Zylinder (6), der eine mit der Absaugquelle (P) verbundene Verbindungsöffnung (6b), eine zur Außenatmosphäre offene Druckablaßöffnung (6a), eine mit dem anderen Ende des Absaugkanals verbundene Öffnung (4a) und ein Ventil (10) mit einem in den Zylinder eingesetzten Körper (10a) und ein in dem Körper (10a) gebildetes Durchgangsloch aufweist, durch das normalerweise die Druckablaßöffnung (6a) mit der Verbindungsöffnung (6b) kommuniziert, dadurch gekennzeichnet, daß der Körper (10a) an dem Zylinder (6) befestigt ist, wobei der Körper (10a) die Verbindung zwischen der Öffnung (4a) und der Ablaßöffnung (6a) und zwischen der Öffnung (4a) und der Verbindungsöffnung (6b) verschließt, wenn die Druckablaßöffnung (6a) mit der Atmosphäre kommuniziert, der Körper (10a) aber aufgrund eines von der Absaugquelle in dem Zylinder (6) erzeugten Unterdrucks elastisch verformt wird, so daß die Öffnung (4a) mit dem Absaugkanal kommuniziert, wenn die Druckablaßöffnung (6a) von der Atmosphäre abgeschlossen ist.

2. Endoskop nach Anspruch 1, in dem der Körper (10a) des Ventils (10) ein Durchgangsloch und eine das Durchgangsloch begrenzende, umgebende Wandung aufweist, von zylindrischer Form ist und konzentrisch und lösbar in dem Zylinder (6) befestigt ist.

3. Endoskop nach Anspruch 2, in dem der zylindrische Körper (10a) des Ventils (10) einen Außendurchmesser aufweist, der im wesentlichen dem Innendurchmesser des Zylinders (6) entspricht und der zylindrische Körper (10) mit seiner Außenfläche eng an der Innenfläche des Zylinders anliegt.

4. Endoskop nach Anspruch 3, in dem der Zylinder (6) an seinem einen Ende die Druckablaßöffnung (6a) und an seinem anderen Ende die Verbindungsöffnung (6b) und eine umgebende Wandung aufweist, durch die die Öffnung gebildet ist, und daß der zylindrische Körper des Ventils so in den Zylinder (6) eingesetzt ist, daß ein Teil der zylindrischen Wand des Körpers die Öffnung (4a) des Zylinders verschließt.

5. Endoskop nach Anspruch 4, in dem wenigstens der Teil des zylindrischen Körpers, der die Öffnung (4a) des Zylinders (6) verschließt, aus elastischem Material gebildet ist.

6. Endoskop nach Anspruch 5, in dem das Ventil (10) Mittel (11) zum Zusammenwirken des Ventils mit dem Zylinder (6) zum Zwecke der Befestigung des Ventils (10) an dem Zylinder (6) aufweist.

7. Endoskop nach Anspruch 6, in dem das Ventil (10) einteilig aus elastischem Material gebildet ist.

8. Endoskop nach Anspruch 7, in dem die zylindrische Wand des zylindrischen Körpers eine gleichmäßige Dicke aufweist.

9. Endoskop nach Anspruch 7, in dem der Teil des zylindrischen Körpers, der die Öffnung (4a) des Zylinders verschließt, dünner ausgebildet ist.

10. Endoskop nach Anspruch 3, in dem der Zylinder (6) eine Druckablaßöffnung (6a) an einem Ende, während sich die Öffnung (4a) an seinem anderen Ende befindet, und eine zylindrische Wand aufweist, durch die die Verbindungs-öffnung gebildet ist, und der zylindrische Körper (10a) eine Bodenwand zum Schließen der Öffnung (4a) des Zylinders und ein Loch in seiner zylindrischen Wand aufweist, um die Verbindungsöffnung (6b) mit der Durchgangsöffnung zu verbinden.

11. Endoskop nach Anspruch 10, in dem die Boden- und zylindrischen Wände des zylindrischen Körpers (10a) einstückig aus elastischem Material gebildet sind.

12. Endoskop nach einem der vorstehenden Ansprüche, in dem der Absaugkanal (4) einen Zweigkanal (4b) aufweist und daß der Zweigkanal (4b) mit einer Öffnung (15) versehen ist, durch die Behandlungswerkzeuge einführbar sind.

13. Endoskop nach Anspruch 12, in dem eine Dichtung (16) in dem Zweigkanal (4b) angeordnet ist, um die Verbindung von dem Absaugkanal mit der Einführöffnung für die Behandlungswerkzeuge für Fluide abzuschließen.

F I G. 1

F I G. 2

F I G. 3

F I G. 4

F I G. 5

0 056 234